(19) Europäisches Patentamt
European Patent Office
Office européen des brevets

(11) Veröffentlichungsnummer : **0 111 934**
**B1**

# (12) EUROPÄISCHE PATENTSCHRIFT

(45) Veröffentlichungstag der Patentschrift : **17.08.88**

(51) Int. Cl.⁴ : **C 07 D 501/46**, A 61 K 31/545

(21) Anmeldenummer : **83112859.0**

(22) Anmeldetag : **21.12.83**

(54) **Cephalosporinderivate und Verfahren zu ihrer Herstellung.**

(30) Priorität : **23.12.82 DE 3247613**

(43) Veröffentlichungstag der Anmeldung : **27.06.84 Patentblatt 84/26**

(45) Bekanntmachung des Hinweises auf die Patenterteilung : **17.08.88 Patentblatt 88/33**

(84) Benannte Vertragsstaaten : **AT BE CH DE FR GB IT LI LU NL SE**

(56) Entgegenhaltungen :
**EP-A- 0 013 762**
**EP-A- 0 064 740**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Dürckheimer, Walter, Dr.**
**Im Lerchenfeld 45**
**D-6234 Hattersheim am Main (DE)**
Erfinder : **Lattrell, Rudolf, Dr.**
**Heuhohlweg 6h**
**D-6240 Königstein/Taunus (DE)**
Erfinder : **Seeger, Karl, Dr.**
**Schwalbenweg 9**
**D-6238 Hofheim am Taunus (DE)**

**EP 0 111 934 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Cephalosporinderivate und Verfahren zu ihrer Herstellung, insbesondere polare Cephemderivate, die in 3-Stellung des Cephemrings durch bestimmte Pyridinium-, Chinolinium- und Isochinolinium-Reste substituiert sind, eine sehr gute antimikrobielle Wirkung gegen gram-positive und gram-negative Bakterien besitzen und deshalb als Arzneimittel zur Behandlung von mikrobiellen Infektionen geeignet sind. Gegenstand der Erfindung sind daher Cephemderivate der allgemeinen Formel I

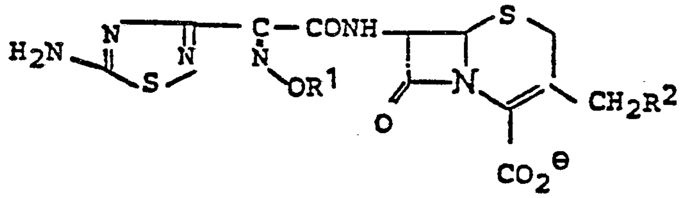

(I)

und deren physiologisch verträgliche Säureadditionssalze, worin bedeuten

$R^1$ $C_1$-$C_2$-Alkyl,

$R^2$ einen Pyridiniumrest

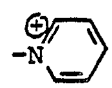
,

der substituiert ist, durch 2 orthoständige Alkylgruppen, die zu einem Tri- bis Pentamethylen-Ring geschlossen sind, in dem ein Ring-C-Atom durch ein Sauerstoffatom ersetzt sein kann, einen 1-Chinolinium- oder einen 2-Isochinoliniumrest und in denen die $R^1$ O-Gruppe in syn-Position steht. Der an den Pyridiniumrest ankondensierte Ring kann, wenn man die beiden benachbarten C-Atome des Pyridiniumrestes bei der Bezeichnung mit berücksichtigt, einen Cyclopenteno-, Cyclohexeno- oder Cyclohepteno-Ring darstellen. Als ein Sauerstoffatom enthaltende, ankondensierte Ringe seien erwähnt : Dihydrofuro und Dihydropyrano.

$R^1$ kann die Bedeutung besitzen von Methyl oder Äthyl.

Ist in den vorstehend genannten ankondensierten Ringsystemen ein C-Atom durch Sauerstoff ersetzt, so kommen insbesondere in Betracht : 2,3- und 3,4-Dihydrofuro, 2,3- und 3,4-Dihydropyrano.

Gegenstand der Erfindung ist weiterhin ein Verfahren zur Herstellung von Verbindungen der Formel I und ihrer physiologisch verträglichen Säureadditionssalze, das dadurch gekennzeichnet ist, daß man

a) eine Verbindung der allgemeinen Formel II

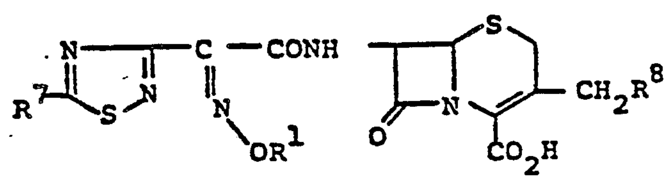

(II)

oder deren Salze oder ein reaktionsfähiges Derivat der Verbindung II, worin $R^1$ die oben genannte Bedeutung hat und $R^7$ eine Amino- oder eine geschützte Aminogruppe bedeutet und $R^8$ eine durch diejenigen Pyridin-, Chinolin- oder Isochinolinderivate, die den Resten $R^2$ der Formel I entsprechen, austauschbare Gruppe bedeutet, mit diesen Pyridin-, Chinolin- oder Isochinolinderivaten umsetzt und

α) eine gegebenenfalls vorhandene Schutzgruppe abspaltet und

β) falls erforderlich, das erhaltene Produkt in ein physiologisch verträgliches Säureadditionssalz überführt, oder

b) eine 7-Amino-cephemverbindung der allgemeinen Formel III

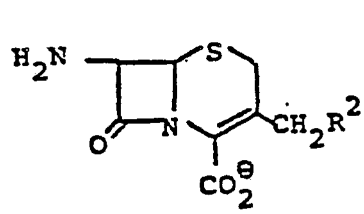

(III)

oder deren Säureadditionssalze, worin $R^2$ die obengenannte Bedeutung hat und wobei die Aminogruppe

auch in Form eines reaktionsfähigen Derivats vorliegen kann, mit einer 2-(5-Amino-1,2,4-thiadiazol-3-yl)-2-syn-oximinoessigsäure der allgemeinen Formel IV,

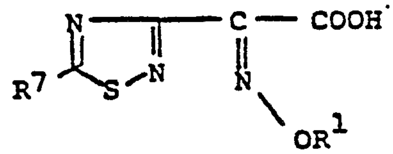

(IV)

worin $R^1$ und $R^7$ die oben genannte Bedeutung besitzen, oder mit einem aktivierten Derivat dieser Verbindung umsetzt und

α) eine gegebenenfalls vorhandene Schutzgruppe abspaltet und

β) falls erforderlich, das erhaltene Produkt in ein physiologisch verträgliches Säureadditionssalz überführt.

Soll die Herstellung der Verbindungen der allgemeinen Formel I durch nucleophilen Austausch von $R^8$ in den Verbindungen der allgemeinen Formel II durch die genannten Pyridin-, Chinolin- oder Isochinolinderivate erfolgen, so kommen als Reste $R^8$ insbesondere in Betracht Acyloxyreste von niederen aliphatischen Carbonsäuren, vorzugsweise mit 1 bis 4 C-Atomen, wie z. B. Acetoxy oder Propionyloxy, insbesondere Acetoxy, die gegebenenfalls substituiert sein können, wie z. B. Chloracetoxy oder Acetylacetoxy. Für $R^8$ kommen auch andere Gruppen in Betracht, wie beispielsweise Halogen, insbesondere Chlor, Brom oder Jod, oder Carbamoyloxy.

Erfindungsgemäß werden bei der nucleophilen Austauschreaktion Ausgangsverbindungen der allgemeinen Formel II, in denen $R^8$ für Acetoxy steht, eingesetzt, oder deren Salze, wie z. B. ein Natrium- oder Kaliumsalz. Die Reaktion wird in einem Lösungsmittel, vorzugsweise in Wasser, oder in einem Gemisch aus Wasser und einem mit Wasser leicht mischbaren organischen Lösungsmittel, wie z. B. Aceton, Dioxan, Acetonitril, Dimethylformamid, Dimethylsulfoxid oder Äthanol, durchgeführt. Die Reaktionstemperatur liegt im allgemeinen im Bereich von etwa 10 bis etwa 100 °C, vorzugsweise zwischen 20 und 80 °C. Die Basenkomponente wird in Mengen zugegeben, die zwischen etwa äquimolaren Mengen und einem bis zu etwa 15-fachen Überschuß liegen. Der Austausch des Restes $R^8$ wird durch Anwesenheit von Neutralsalzionen, vorzugsweise von Jodid- oder Thiocyanationen, im Reaktionsmedium erleichtert. Insbesondere werden etwa 10 bis etwa 80 Äquivalente Kaliumjodid, Natriumjodid, Kaliumthiocyanat oder Natriumthiocyanat zugegeben. Die Reaktion wird vorteilhaft in der Nähe des Neutralpunktes, vorzugsweise bei einem pH-Wert im Bereich von etwa 5 bis etwa 8 durchgeführt.

Liegt die Gruppe $R^7$ als geschützte Aminofunktion vor, so eignen sich als Aminoschutzgruppen z. B. gegebenenfalls substituiertes Alkyl, wie beispielsweise tert.-Butyl, tert.-Amyl, Benzyl, p-Methoxybenzyl, Trityl, Benzhydryl, vorzugsweise Trityl; Trialkylsilyl, wie beispielsweise Trimethylsilyl; gegebenenfalls substituiertes aliphatisches Acyl, wie z. B. Formyl, Chloracetyl, Bromacetyl, Trichloracetyl und Trifluoracetyl, vorzugsweise Formyl; oder gegebenenfalls substituiertes Alkoxycarbonyl, wie beispielsweise Trichloräthoxycarbonyl, Benzyloxycarbonyl oder tert.-Butyloxycarbonyl, vorzugsweise tert.-Butyloxycarbonyl und Benzyloxycarbonyl; oder Dimethylaminomethylen.

Die Schutzgruppe kann nach der Austauschreaktion in an sich bekannter Weise abgespalten werden, z. B. die Tritylgruppe mittels einer Carbonsäure, wie z. B. Essigsäure, Trifluoressigsäure, Ameisensäure, oder die Benzyloxycarbonylgruppe hydrogenolytisch.

Die Reaktionsprodukte der Formel I können aus der Reaktionsmichung in üblicher Weise, z. B. durch Gefriertrocknen der Wasserphase, Chromatographie oder auch durch Ausfällen als schwerlösliches Salz, beispielsweise als Hydrojodid- oder Hydrothiocyanatsalz, isoliert werden.

Die nucleophile Austauschreaktion an Verbindungen der allgemeinen Formel II kann auch so erfolgen, daß die Reaktion in Gegenwart der den Resten $R^2$ entsprechenden Basenkomponente und von Trimethyljodsilan vorgenommen wird. Diese Variante der Austauschreaktion wird vorteilhafterweise so durchgeführt, daß zu einem Gemisch der Verbindung II und der Base in einem geeigneten Lösungsmittel Trimethyljodsilan zugegeben wird. Es kann aber auch so verfahren werden, daß die Verbindung zuerst mit Trimethyljodsilan nach den unten genannten Reaktionsbedingungen zur Reaktion gebracht wird und sodann die Base zugegeben wird.

Geeignete Lösungsmittel sind chlorierte Kohlenwasserstoffe, wie Methylenchlorid, Chloroform, Dichloräthan, Trichloräthan, Tetrachlorkohlenstoff, oder Niederalkylnitrile, wie Acetonitril oder Propionitril.

Die Base wird in mindestens stöchiometrischer Menge bis zu einem zwanzigfachen Überschuß zugegeben, vorzugsweise wird mit einem fünf- bis fünfzehnfachen Überschuß gearbeitet.

Trimethyljodsilan wird ebenfalls in mindestens stöchiometrischer Menge bis zu einem zwanzigfachen Überschuß, vorzugsweise in einem fünf- bis fünfzehnfachen Überschuß zugegeben.

Die Reaktion wird bei Temperaturen zwischen —5 ° und + 100 °C, vorzugsweise zwischen 10 ° und 80 °C ausgeführt.

Die Reaktionsprodukte der Formel I können nach Hydrolyse der Reaktionsmischung durch Zugabe von Wasser oder wässrigen Mineralsäuren, z. B. verdünnter HCl, HBr, HJ oder $H_2SO_4$, aus der wässrigen

Phase in üblicher Weise, z. B. durch Gefriertrocknen der Wasserphase, Chromatographie und dergleichen isoliert werden. Vorzugsweise werden die polaren Reaktionsprodukte aus der wässrigen Lösung in Form eines schwerlöslichen Salzes, beispielsweise nach Zugabe von KSCN oder KJ, als Hydrothiocyanat- oder Hydrojodidsalz ausgefällt.

Für den Fall, daß $R^8$ für eine Carbamoyloxygruppe steht, wird die Austauschreaktion analog durchgeführt.

Steht $R^8$ für Halogen, insbesondere Brom oder Jod, so erfolgt der Austausch in literaturbekannter Weise. Liegt die Verbindung II dabei in Form eines reaktionsfähigen Derivates vor, so kommen beispielsweise Silylderivate in Betracht, die bei der Umsetzung von Verbindungen der allgemeinen Formell II mit einer Silylverbindung gebildet werden, wie z. B. Trimethylchorsilan oder Bis-(trimethylsilyl) acetamid. In diesem Fall wird die Umsetzung zweckmäßig in Gegenwart eines Lösungsmittel, wie Methylenchlorid oder Acetonitril, vorgenommen.

Die Acylierung der Verbindungen der allgemeinen Formel III oder von deren Additionssalzen, beispielsweise mit Chlorwasserstoffsäure, Bromwasserstoffsäure, Salpetersäure, Schwefelsäure, Phosphorsäure, oder einer organischen Säure, wie z. B. Methansulfonsäure, p-Toluolsulfonsäure oder Maleinsäure, wird mit Carbonsäuren der allgemeinen Formel IV oder mit einem reaktionsfähigen Derivat einer solchen Säure durchgeführt. In manchen Fällen ist es dabei von Vorteil, die 5-Aminogruppe in den Verbindungen der allgemeinen Formel IV vor der Umsetzung zu schützen. Als Aminoschutzgruppen eignen sich die vorstehend für $R_7$ beschriebenen Schutzgruppen. Die Schutzgruppe kann nach der Acylierung in an sich bekannter Weise abgespalten werden, z. B. die Tritylgruppe mittels einer Carbonsäure, wie z. B. Ameisensäure oder Trifluoressigsäure, oder die Chloracetylgruppe mittels Thioharnstoff.

Werden die Carbonsäuren der allgemeinen Formel IV sowie ihre an der Aminogruppe geschützten Derivate selbst als Acylierungsmittel eingesetzt, so wird zweckmäßig in Gegenwart eines Kondensationsmittels, beispielsweise eines Carbodiimids, wie beispielsweise N,N'-Dicyclohexylcarbodiimid, gearbeitet.

Die Aktivierung von Carbonsäuren der allgemeinen Formel IV kann in besonders günstiger Weise durch Behandlung mit bestimmten Carbonsäureamiden und beispielsweise Phosgen, Phosphorpentachlorid, Tosylchlorid, Thionylchlorid oder Oxalylchlorid erfolgen, wie sie z. B. in der deutschen Patentschrift 28 04 040 beschrieben wird.

Als aktivierte Derivate der Carbonsäuren der allgemeinen Formel IV eignen sich insbesondere auch Halogenide, vorzugsweise Chloride, die in an sich bekannter Weise durch Behandlung mit Halogenierungsmittel, wie z. B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel IV eignen sich ferner die Anhydride und gemischten Anhydride, Azide, aktivierten Ester und Thioester, vorzugsweise mit p-Nitrophenol, 2,4-Dinitrophenol, Methylencyanhydrin, N-Hydroxysuccinimid und N-Hydroxyphthalimid, insbesondere diejenigen mit 1-Hydroxybenzotriazol, 6-Chlor-1-hydroxybenzotriazol und 2-Mercaptobenzthiazol. Als gemischte Anhydride sind besonders geeignet solche mit niederen Alkansäuren, wie z. B. Essigsäure, und besonders bevorzugt solche mit substituierten Essigsäuren, wie z. B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäuren der Formel IV, in denen die Aminogruppe geschützt ist, mit Chlorameisensäure-benzylester, -p-nitrobenzylester, -iso-butylester, -ethylester oder -allylester gewinnt. Die aktivierten Derivate können als isolierte Substanzen, aber auch in situ umgesetzt werden.

Im allgemeinen erfolgt die Umsetzung der Cephemderivate der allgemeinen Formel III mit einer Carbonsäure der allgemeinen Formel IV oder einem aktivierten Derivat derselben in Gegenwart eines inerten Lösungsmittels. Insbesondere eignen sich chlorierte Kohlenwasserstoffe, wie vorzugsweise Methylenchlorid und Chloroform ; Äther, wie z. B. Diäthyläther, vorzugsweise Tetrahydrofuran und Dioxan ; Ketone, wie vorzugsweise Aceton und Butanon ; Amide, wie vorzugsweise Dimethylformamid und Dimethylacetamid, oder Pyridin. Es kann sich auch als vorteilhaft erweisen, Gemische der genannten Lösungsmittel zu verwenden. Dies ist oftmals dann der Fall, wenn die Cephemverbindung der allgemeinen Formel III mit einem in situ erzeugten aktivierten Derivat einer Carbonsäure der Formel IV umgesetzt wird.

Die Umsetzung von Cephemverbindungen der Formel III mit Carbonsäuren der Formel IV bzw. deren aktivierten Derivaten kann in einem Temperaturbereich von etwa — 80 bis etwa + 80 °C, vorzugsweise zwischen — 30 und + 50 °C, insbesondere jedoch zwischen etwa — 20 °C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen etwa 1/4 und etwa 72 Stunden.

Die Reaktion mit Säurehalogeniden kann gegebenenfalls in Gegenwart eines säurebindenden Mittels zur Bindung des freigesetzten Halogenwasserstoffs durchgeführt werden. Als solche eignen sich insbesondere tertiäre Amine, wie z. B. Triäthylamin, Dimethylanilin oder Pyridin, anorganische Basen, wie z. B. Kaliumcarbonat oder Natriumcarbonat, Alkylenoxide, wie z. B. Propylenoxid. Auch die Anwesenheit eines Katalysators, wie z. B. von Dimethylaminopyridin, kann gegebenenfalls von Vorteil sein.

Liegt in den Verbindungen der allgemeinen Formel III die Aminogruppe in Form eines reaktionsfähigen Derivats vor, so kann es sich um ein solches handeln, wie es aus der Literatur für Amidierungen bekannt ist. So kommen beispielsweise Silylderivate in Betracht, die bei der Umsetzung von Verbindungen der allgemeinen Formel III mit einer Silylverbindung gebildet werden, wie z. B. Trimethylchlorsilan oder Bis-(trimethylsilyl)-acetamid. Wird die Umsetzung mit einer solchen, an der Aminogruppe aktivierten Verbindung durchgeführt, so ist es zweckmäßig, die Reaktion in einem inerten Lösungsmittel, wie z. B. Methylenchlorid, Tetrahydrofuran oder Dimethylformamid, durchzuführen.

Als physiologisch verträgliche Säureadditionssalze der Verbindungen der allgemeinen Formel I seien beispielsweise erwähnt solche mit Chlorwasserstoffsäure, Bromwasserstoffsäure, Salpetersäure, Phosphorsäure, Schwefelsäure oder organischen Säuren, wie z. B. Methansulfonsäure, p-Toluolsulfonsäure oder Maleinsäure.

Die Verbindungen der allgemeinen Formel III können in an sich bekannter Weise, beispielsweise aus der 7-Aminocephalosporansäure oder an der Aminogruppe geschützter 7-Aminocephalosporansäure auf dieselbe Weise erhalten werden, die sie vorstehend für den nucleophilen Austausch von $R^8$ beschrieben wurde.

Die Verbindungen der allgemeinen Formel IV sowie die den Resten $R^2$ entsprechenden Pyridin-, Chinolin- und isochinolinbasen sind literaturbekannt oder nach literaturbekannten Verfahren herstellbar.

Die erfindungsgemäß erhaltenen Verbindungen der allgemeinen Formel I und ihre physiologisch verträglichen Säureadditionssalze zeigen bemerkenswert gute antibakterielle Wirksamkeit sowohl gegen grampositive als auch gramnegative bakterielle Keime.

Auch gegen penicillinase- und cephalosporinase-bildende Bakterien sind die Verbindungen der Formel I unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch einen Gehalt an einer oder mehreren der erfindungsgemäßen Verbindungen charakterisiert sind.

Die erfindungsgemäßen Produkte können auch in Kombination mit anderen Wirkstoffen, beispielsweise aus der Reihe der Penicilline, Cephalosporine oder Aminoglykoside zur Anwendung kommen.

Die Verbindungen der allgemeinen Formel I und ihre physiologisch verträglichen Säureadditionssalze können oral, intramuskulär oder intravenös verabfolgt werden. Arzneipräparate, die eine oder mehrere Verbindungen der allgemeinen Formel I als Wirkstoff enthalten, können hergestellt werden, indem man die Verbindungen der Formel I mit mehreren pharmakologisch verträglichen Trägerstoffen oder Verdünnungsmitteln, wie z. B. Füllstoffen, Emulgatoren, Gleitstoffen, Geschmackskorrigentien, Farbstoffen oder Puffersubstanzen vermischt und in eine geeignete galenische Zubereitungsform bringt, wie beispielsweise Tabletten, Dragees, Kapseln, oder eine zur parenteralen Applikation geeignete Suspension oder Lösung.

Als Träger- oder Verdünnungsmittel seien beispielsweise erwähnt Traganth, Milchzucker, Talkum, Agar-Agar, Polyglykole, Äthanol und Wasser. Puffersubstanzen sind beispielsweise organische Verbindungen, wie z. B. N,N'-Dibenzyläthylendiamin, Diäthanolamin, Äthylendiamin, N-Methylglucamin, N-Benzylphenäthylamin, Diäthylamin, Tris (hydroxymethyl) aminomethan, oder anorganische Verbindungen, wie z. B. Phosphatpuffer, Natriumbicarbonat, Natriumcarbonat. Für die parenterale Applikation kommen vorzugsweise Suspensionen oder Lösungen in Wasser mit oder ohne Puffersubstanzen in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindungen der allgemeinen Formel I oder ihrer physiologisch verträglichen Säureadditionssalze liegen bei etwa 0,4 bis 20 g/Tag, vorzugsweise bei 0,5 bis 4 g/Tag, für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg, vorzugsweise von etwa 100 bis 500 mg, enthalten kann.

Aus der EP-A-0 064 740 sind antimikrobiell wirksame Cephalosporinderivate bekannt, die sich von den anmeldungsgemäßen Verbindungen durch Ersatz der 5-Amino-1,2,4-thiadiazolgruppe durch einen 5-Amino-1,3-thiazolrest unterscheiden. Es ist weiterhin aus der EP-A-0 013 762 bekannt, daß ein Pyridiniummethylrest als Substituent in 3-Stellung eines einen 1,2,4-Thiadiazolring enthaltenden Cephemderivats auftreten kann.

Die folgenden Ausführungsbeispiele für erfindungsgemäß herstellbare syn-Verbindungen dienen zur weiteren Erläuterung der Erfindung, schränken sie jedoch nicht darauf ein.

## Beispiel 1

3-[(2,3-Cyclopenteno-1-pyridinium) methyl]-7-[2-syn-methoxyimino-2-(5-amino-1,2,4-thiadiazol-3-yl) acetamido]-ceph-3-em-4-carboxylat

a)

α) Ein Gemisch aus 0,57 g (1 mMol) 7-[2-(5-Amino-1,2,4-thiadiazol-3-yl)-2-syn-methoxyimino-acetamido]-cephalosporansäure-trifluoroacetat, 30 mg Ascorbinsäure, 6,65 g (40 mMol) Kaliumjodid, 1,8 ml (15 mMol) 2,3-Cyclopentenopyridin, 7 ml Wasser und 3 ml Aceton wird 4 Stunden bei 65-67 °C unter

Rühren erhitzt.

Nach dem Erkalten wird die dunkelgefärbte Lösung an Kieselgel (« Lobar-C® »-Säule-Merck) mit Acetan : Wasser (2 : 1) chromatographiert.

Die Produktfraktionen werden eingeengt und gefriergetrocknet. Man erhält 0,14 g (27 % d. Th.) eines farblosen amorphen Feststoffs.

IR (KBr) : 1 770 $cm^{-1}$ (Lactam-CO)

$^1$H-NMR ($CF_3CO_2D$ : $\delta$ = 2,20-2,80 (m, 2H, Cyclopenten-H ; 3,10-4,05 (m, 6H, 4 Cyclopenten-H und $SCH_2$) ; 4,30 (s, 3H, $OCH_3$) ; 5,20-6,25 (m, 4H, 3-$CH_2$ und 2 Lactam-H) ; 7,66-8,70 ppm (m, 3H, Py).

β) Zu einer Mischung aus 0,57 g (1 mMol) 7-[2-(5-Amino-1,2,4-thiadiazol-3-yl)-2-syn-methoxyimino-acetamido]-cephalosporansäure-trifluoroacetat und 1,07 g (9 mMol) 2,3-Cyclopentenopyridin in 10 ml Methylenchlorid werden bei 5 °C 1,4 g (7 mMol) Trimethyljodsilan gegeben und sodann 2 Stunden unter Rückfluß erhitzt. Nach dem Erkalten werden 7 ml 2 n-HCl zugegeben, 10 Minuten bei ca. 15 °C gerührt und durch Zugabe von festem Kaliumhydrogencarbonat auf pH 6,5 gestellt. Die wässrige Phase wird abgetrennt und über eine « Lobar-C® »-Säule mit Aceton : Wasser (2 : 1) chromatographiert. Nach dem Gefriertrocknen der Produktfraktionen erhält man 0,29 g (56 % d. Th) eines farblosen Feststoffs, der in allen Eigenschaften mit dem vorstehend beschriebenen identisch ist.

b)

404 mg (2 mMol) 2-(5-Amino-1,2,4-thiadiazol-3-yl)-2-syn-methoxyimino-essigsäure werden in 6 ml N,N-Dimethylformamid gelöst. Nach Zugabe von 280 mg (2,1 mMol (1-Hydroxybenztriazolhydrat und 410 mg (2 mMol) N,N'-Dicyclohexylcarbodiimid wird 2 Stunden bei Raumtemperatur gerührt. Vom ausgefallenen Dicyclohexylharnstoff wird filtriert und zum Filtrat eine Lösung von 808 mg (2 mMol) 7-Amino-3-[(2,3-cyclopenteno-1-pyridinium) methyl]-ceph-3-em-4-carboxylat-Dihydrochlorid in 10 ml N,N-Dimethylformamid und 1 ml Wasser gegeben. Der Ansatz wird 3 Stunden bei Raumtemperatur gerührt, im Vakuum eingeengt und der Rückstand in 10 ml Wasser gelöst. Von wenig Ungelöstem wird filtriert und die Lösung über eine « Lobar-C® »-Säule mit Aceton : Wasser (2 : 1) chromatographiert.

Die Produktfraktionen werden gefriergetrocknet und man erhält 580 mg (56 % d. Th.) der Titelverbindung als amorphen Feststoff.

Er ist in allen Eigenschaften mit der vorstehend beschriebenen Verbindung identisch.

Analog Beispiel 1 werden die nachfolgend aufgeführten Verbindungen als amorphe Feststoffe erhalten, die der allgemeinen Formel I mit $R^1$ = Methyl entsprechen und als Rest $R^2$ den in der zweiten Spalte der Tabelle 1 angegebenen Substituenten tragen.

Tabelle 1

| Beispiel | $R^2$ | $^1$H-NMR in $CF_3CO_2D$; $\delta$ (ppm) |
|---|---|---|
| 2 | 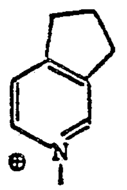 | 2.2-3.9 (m, 8H, 6 Cyclopenten-H, $SCH_2$); 4.27 (s, 3H, $OCH_3$); 5.10-6.30 (m, 4H, 3-$CH_2$ und 2 Lactam-H); 7.7-8.8 ppm (m, 3H, Py) |
| 3 | 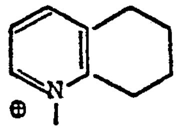 | 1.7-2.4 (m, 4 Cyclohexen-H); 2.8-4.0 (m, 6H, 4 Cyclohexen-H und $SCH_2$); 4.30 (s, 3H, $OCH_3$); 5.15-6.20 (m, 4H, 3-$CH_2$ und 2 Lactam-H); 7.55-8.68 ppm (m, 3H, Py) |
| 4 | 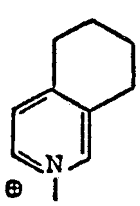 | 1.8-2.35 (m, 4 Cyclohexen-H; 2.7-3.9 (m, 6H, 4 Cyclohexen-H und $SCH_2$); 4.28 (s, 3H, $OCH_3$); 5.10-6.25 (m, 4H, 3-$CH_2$ und 2 Lactam-H); 7.6-8.7 (m, 3H, Py) |

Tabelle 1 (Fortsetzung)

| Beispiel | $R^2$ | $^1H$-NMR in $CF_3CO_2D$: $\delta$ (ppm) |
|---|---|---|
| 5 | 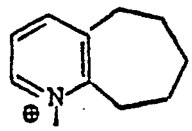 | 1.6-2.4 (m, 6 Cyclohepten-H); 3.0-4.0 (m, 6H, 4 Cyclohepten-H und $SCH_2$); 4.30 (s, 3H, $OCH_3$); 5.20-6.35 (m, 4H, 3-$CH_2$ und 2 Lactam-H); 7.6-8.7 ppm (m, 3H, Py) |
| 6 | 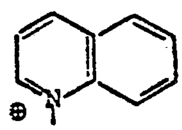 | 3.43 und 3.90 (AB, J=19Hz, 2H, $SCH_2$); 4.30 (s, 3H, $OCH_3$); 5.25-6.40 (m, 4H, 3-$CH_2$ und 2 Lactam-H); 7.90-8.60 (m, 5H, Chinolin-H); 8.90-9.40 ppm (m, 2H, Chinolin-H) |
| 7 | 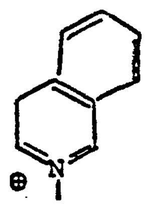 | 3.41 und 3.92 (AB, J=19Hz, 2H, $SCH_2$); 4.28 (s, 3H, $OCH_3$); 5.26-6.30 (m, 4H, 3-$CH_2$ und 2 Lactam-H mit je 1 d bei 5.42 und 6.11, J=5Hz, C-6 bzw. C-7-H); 8.0-8.8 (m, 6H, Isochinolin-H); 9.78 ppm (bs, 1H, Isochinolin-H) |

Analog Beispiel 1 werden die nachfolgend aufgeführten Verbindungen als amorphe Feststoffe erhalten, die der allgemeinen Formel I' ($R^2$ = 2,3 Cyclopenteno-1-pyridinium) entsprechen und als Rest $R^1$ den in der zweiten Spalte der Tabelle 2 angegebenen Substituenten tragen.

Tabelle 2

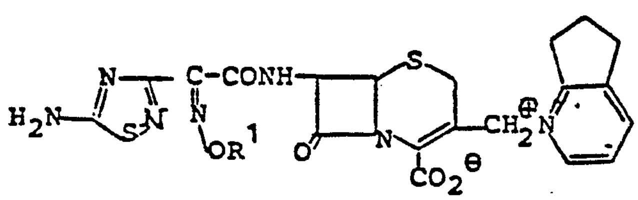

I'

| Beispiel | $R^1$ | $^1H$-NMR in $CF_3CO_2D$: $\delta$ (ppm) |
|---|---|---|
| 8 | $C_2H_5$ | 1.42 (t,J=7Hz, 3H, $CH_2CH_3$); 2.15-2.80 m, 2H, Cyclopenten-H); 3.1-4.0 (m, 6H, 4 Cyclopenten-H und $SCH_2$); 4.48 (q,J=7Hz, 2H, $CH_2CH_3$); 5.15-6.35 (m, 4H, 3-$CH_2$ und 2 Lactam-H); 7.65-8.70 ppm (m, 3H, Py) |

Analog Beispiel 1 wird die nachfolgend aufgeführte Verbindung als amorpher Feststoff erhalten, die der allgemeinen Formel I mit $R^1$ = Methyl entspricht und als Rest $R^2$ den in der zweiten Spalte der folgenden Tabelle angegebenen Substituenten trägt.

| Beispiel | $R^2$ | $^1H$-NMR in $CF_3CO_2D$: (ppm) |
|---|---|---|
| 9 | 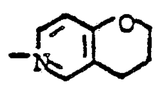 | 1.9–2.45 (m, 2H, Pyran-H); 2.65–3.25 (m, 2H, Pyran-H); 3.42 und 3.75 (AB, J = 19 Hz, 2 H, $SCH_2$); 4.26 (s, 3H, $OCH_3$); 4.4–4.8 (m, 2H, Pyran-H); 4.85–6.20 (m, 4H, $CH_2Py$ und 2 Lactam-4); 7,25 (d, J = 7 Hz, Py-H); 8.2–8.6 (m, 2H, Py) |

**Patentansprüche** (für die Vertragsstaaten : BE, CH, DE, FR, GB, IT, LU, LI, NL, SE)

1. Cephemderivate der allgemeinen Formel I

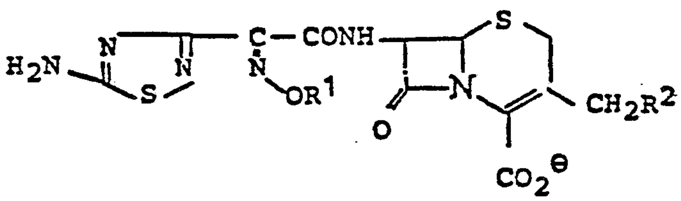

(I)

und deren physiologisch verträgliche Säureadditionssalze, worin bedeuten

$R^1$ $C_1$-$C_2$-Alkyl,

$R^2$ einen Pyridiniumrest

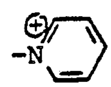 ,

der substituiert ist, durch 2 orthoständige Alkylgruppen, die zu einem Tri- bis Pentamethylen-Ring geschlossen sind, in dem ein C-Atom durch ein Sauerstoffatom ersetzt sein kann, einen 1-Chinolinium- oder einen 2-Isochinoliniumrest

und in denen die $R^1$ O-Gruppe in syn-Position steht.

2. Verfahren zur Herstellung von Verbindungen der Formel I und ihrer physiologisch verträglichen Säureadditionssalze, das dadurch gekennzeichnet ist, daß man

a) eine Verbindung der allgemeinen Formel II

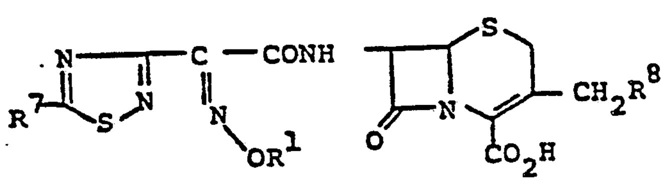

(II)

oder deren Salze oder ein reaktionsfähiges Derivat der Verbindung II, worin $R^1$ die oben genannte Bedeutung hat und $R^7$ eine Amino- oder eine geschützte Aminogruppe bedeutet und $R^8$ eine durch diejenigen Pyridin-, Chinolin- oder Isochinolinderivate, die den Resten $R^2$ der Formel I entsprechen, austauschbare Gruppe bedeutet, mit diesen Pyridin-, Chinolin- oder Isochinolinderivaten umsetzt und

α) eine gegebenenfalls vorhandene Schutzgruppe abspaltet und

β) falls erforderlich, das erhaltene Produkt in ein physiologisch verträgliches Säureadditionssalz überführt, oder

b) eine 7-Amino-cephemverbindung der allgemeinen Formel III

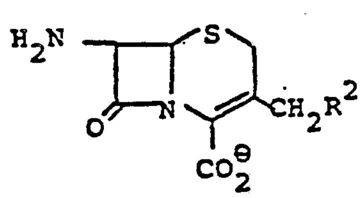

(III)

oder deren Säureadditionssalze, worin $R^2$ die obengenannte Bedeutung hat und wobei die Aminogruppe auch in Form eines reaktionsfähigen Derivats vorliegen kann, mit einer 2-(5-Amino-1,2,4-thiadiazol-3-yl)-2-syn-oximinoessigsäure der allgemeinen Formel IV,

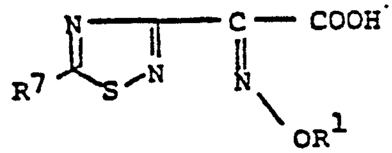

(IV)

worin $R^1$ und $R^7$ die oben genannte Bedeutung besitzen, oder mit einem aktivierten Derivat dieser Verbindung umsetzt und

α) eine gegebenenfalls vorhandene Schutzgruppe abspaltet und

β) falls erforderlich, das erhaltene Produkt in ein physiologisch verträgliches Säureadditionssalz überführt.

3. Verfahren gemäß Anspruch 2, dadurch gekennzeichnet, daß der nucleophile Austausch des Substituenten $R^8$ in Gegenwart von Neutralsalzionen, insbesondere von Jodid oder Thiocyanationen erfolgt.

4. Verfahren gemäß Anspruch 2, dadurch gekennzeichnet, daß der nucleophile Austausch des Substituenten $R^8$ in Gegenwart der dem Rest $R^2$ zugrundeliegenden Base und von Trimethyljodsilan erfolgt.

5. Gegen bakterielle Infektionen wirksame pharmazeutische Präparate, gekennzeichnet durch einen Gehalt an Cephemderivaten der allgemeinen Formel I.

6. Verfahren zur Herstellung von gegen bakterielle Infektionen wirksamen pharmazeutischen Präparaten, dadurch gekennzeichnet, daß ein Cephemderivat der allgemeinen Formel I gegebenenfalls mit pharmazeutisch üblichen Trägerstoffen, Verdünnungsmitteln oder Puffersubstanzen in eine pharmazeutisch geeignete Verabreichungsform gebracht wird.

7. Cephemderivat der allgemeinen Formel I gemäß Anspruch 1 zur Bekämpfung bakterieller Infektionen.

**Patentansprüche** (für den Vertragsstaat AT)

1. Verfahren zur Herstellung von Cephemderivaten der Formel I

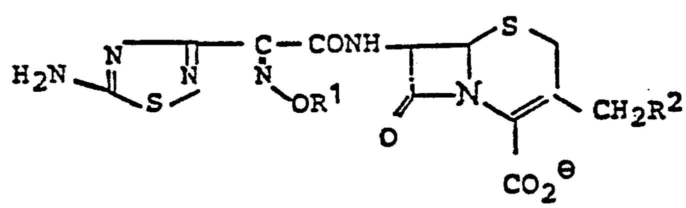

(I)

und deren physiologisch verträgliche Säureadditionssalze, worin bedeuten

$R^1$ $C_1$-$C_2$-Alkyl,

$R^2$ einen Pyridiniumrest

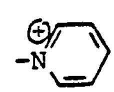 ,

der substituiert ist, durch 2 orthoständige Alkylgruppen, die zu einem Tri- bis Pentamethylen-Ring geschlossen sind, in dem ein C-Atom durch ein Sauerstoffatom ersetzt sein kann, einen 1-Chinolinium- oder einen 2-Isochinoliniumrest,

in der die $R^1$ O-Gruppe in syn-Position steht, dadurch gekennzeichnet, daß man

a) eine Verbindung der allgemeinen Formel II

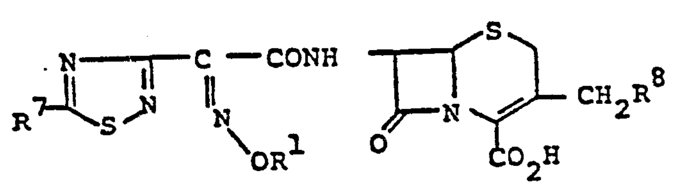

(II)

oder deren Salze oder ein reaktionsfähiges Derivat der Verbindung II, worin $R^1$ die oben genannte Bedeutung hat und $R^7$ eine Amino- oder eine geschützte Aminogruppe bedeutet und $R^8$ eine durch

diejenigen Pyridin-, Chinolin- oder Isochinolinderivate, die den Resten $R^2$ der Formel I entsprechen, austauschbare Gruppen bedeutet, mit diesen Pyridin-, Chinolin-, oder Isochinolinderivaten umsetzt und

α) eine gegebenenfalls vorhandene Schutzgruppe abspaltet und

β) falls erforderlich, das erhaltene Produkt in ein physiologisch verträgliches Säureadditionssalz überführt, oder

b) eine 7-Amino-cephemverbindung der allgemeinen Formel III

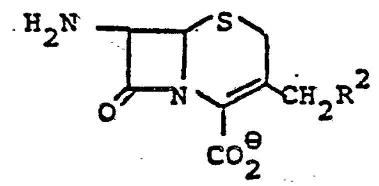

(III)

oder deren Säureadditionssalze, worin $R^2$ die obengenannte Bedeutung hat und wobei die Aminogruppe auch in Form eines reaktionsfähigen Derivats vorliegen kann, mit einer 2-(5-Amino-1,2,4-thiadiazol-3-yl)-2-syn-oximinoessigsäure der allgemeinen Formel IV,

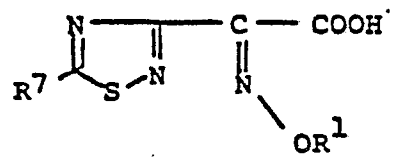

(IV)

worin $R^1$ und $R^7$ die oben genannte Bedeutung besitzen, oder mit einem aktivierten Derivat dieser Verbindung umsetzt und

α) eine gegebenenfalls vorhandene Schutzgruppe abspaltet und

β) falls erforderlich, das erhaltene Produkt in ein physiologisch verträgliches Säureadditionssalz überführt.

2. Verfahren gemäß Anspruch 2, dadurch gekennzeichnet, daß der nucleophile Austausch des Substituenten $R^8$ in Gegenwart von Neutralsalzionen, insbesondere von Jodid oder Thiocyanationen erfolgt.

3. Verfahren gemäß Anspruch 2, dadurch gekennzeichnet, daß der nucleophile Austausch des Substituenten $R^8$ in Gegenwart der dem Rest $R^2$ zugrundeliegenden Base und von Trimethyljodsilan erfolgt.

**Claims** (for the Contracting States : BE, CH, DE, FR, GB, IT, LU, LI, NL, SE)

1. A cephem derivative of the formula I

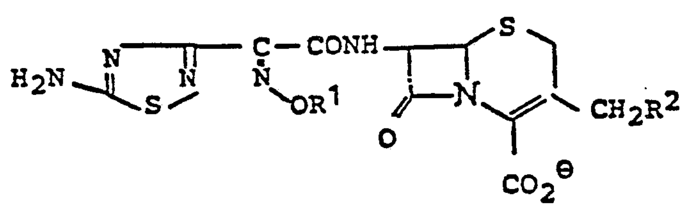

(I)

and physiologically acceptable acid addition salts thereof in which

$R^1$ denotes $C_1$-$C_2$-alkyl,

$R^2$ denotes a pyridinium radical

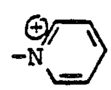 ,

which is substituted by 2 alkyl groups in the ortho-position which are linked to form a trimethylene to pentamethylene ring in which one carbon atom can be replaced by an oxygen atom, or denotes a 1-quinolinium radical or a 2-isoquinolinium radical and in which the $R^1O$ group is in the syn-position.

2. A process for the preparation of a compound of the formula I and its physiologically acceptable acid addition salts, which comprises

a) reacting a compound of the general formula II

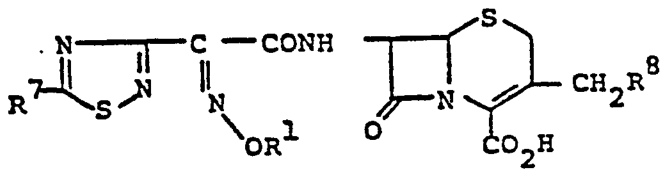

(II)

or salts thereof or a reactive derivative of the compound II in which $R^1$ has the meaning mentioned above and $R^7$ denotes an amino group or a protected amino group and $R^8$ denotes a group which can be replaced by the pyridine, quinoline or isoquinoline derivatives corresponding to the radicals $R^2$ of the formula I, with these pyridine, quinoline or isoquinoline derivatives, and

α) splitting off a protective group which may be present and

β) if necessary, converting the resulting product into a physiologically acceptable acid·addition salt, or

b) reacting a 7-aminocephem compound of the general formula III

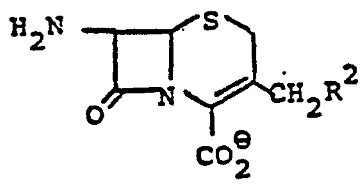

(III)

or acid addition salts thereof in which $R^2$ has the above-mentionned meaning and in which the amino group can also be present in the form of a reactive derivative, with a 2-(5-amino-1,2,4-thiadiazol-3-yl)-2-syn-oximinoacetic acid of the general formula IV

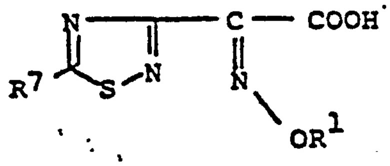

(IV)

in which $R^1$ and $R^7$ have the meaning mentioned above, or with an activated derivative of this compound, and

α) splitting off a protective group which may be present, and

β) if necessary, converting the resulting product into a physiologically acceptable acid addition salt.

3. The process as claimed in claim 2, wherein the nucleophilic replacement of the substituent $R^8$ is effected in the presence of neutral salt ions, in particular iodide or thiocyanate ions.

4. A process as claimed in claim 2, wherein the nucleophilic replacement of the substituent $R^8$ is effected in the presence of the base from which the radical $R^2$ is derived, and of trimethyliodosilane.

5. A pharmaceutical formulation which is effective against bacterial infections and contains cephem derivatives of the general formula I.

6. A process for the preparation of a pharmaceutical formulation which is effective against bacterial infections, which comprises bringing a cephem derivative of the general formula I into a pharmaceutically suitable administration form, if appropriate together with pharmaceutically customary excipients, diluents or buffer substances.

7. The use of cephem derivatives of the general formula I as claimed in claim 1 for combating bacterial infections.

**Claims** (for the Contracting State AT)

1. A process for the preparation of a cephem derivative of the formula I

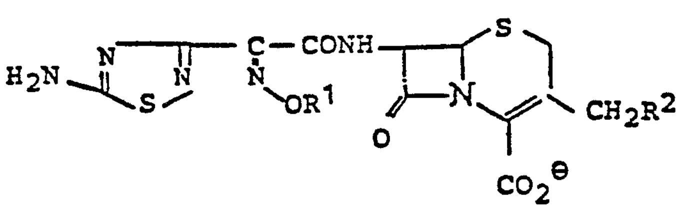

(I)

and physiologically acceptable acid addition salts thereof in which

$R^1$ denotes $C_1$-$C_2$-alkyl,

$R^2$ denotes a pyridinium radical

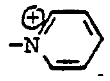,

which is substituted by 2 alkyl groups in the ortho-position which are linked to form a trimethylene to pentamethylene ring in which one carbon atom can be replaced by an oxygen atom, or denotes a 1-quinolinium radical or a 2-isoquinolinium radical in which the $R^1O$ group is in the syn-position which comprises

a) reacting a compound of the general formula II

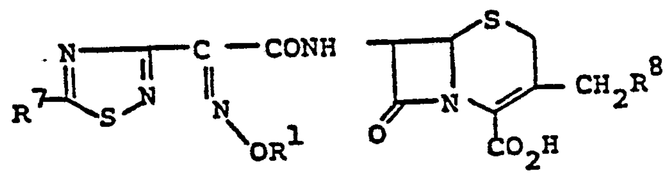

(II)

or salts thereof or a reactive derivative of the compound II in which $R^1$ has the meaning mentioned above and $R^7$ denotes an amino group or a protected amino group and $R^8$ denotes a group which can be replaced by the pyridine, quinoline or isoquinoline derivatives corresponding to the radicals $R^2$ of the formula I, with these pyridine, quinoline or isoquinoline derivatives, and

α) splitting off a protective group which may be present and

β) if necessary, converting the resulting product into a physiologically acceptable acid addition salt, or

b) reacting a 7-aminocephem compound of the general formula III

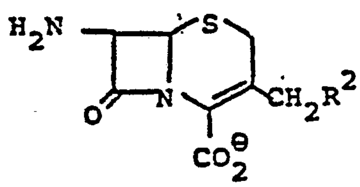

(III)

or acid addition salts thereof in which $R^2$ has the abovementioned meaning and in which the amino group can also be present in the form of a reactive derivative, with a 2-(5-amino-1,2,4-thiadiazol-3-yl)-2-syn-oximinoacetic acid of the general formula IV

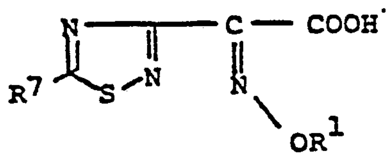

(IV)

in which $R^1$ and $R^7$ have the meaning mentioned above, or with an activated derivative of this compound, and

α) splitting off a protective group which may be present, and

β) if necessary, converting the resulting product into a physiologically acceptable acid addition salt.

2. The process as claimed in claim 1, wherein the nucleophilic replacement of the substituent $R^8$ is effected in the presence of neutral salt ions, in particular iodide or thiocyanate ions.

3. The process as claimed in claim 1, wherein the nucleophilic replacement of the substituent $R^8$ is effected in the presence of the base from which the radical $R^2$ is derived, and of trimethyliodosilane.

**Revendications** (pour les Etats contractants : BE, CH, DE, FR, GB, IT, LU, LI, NL, SE)

1. Dérivés céphème de formule générale I

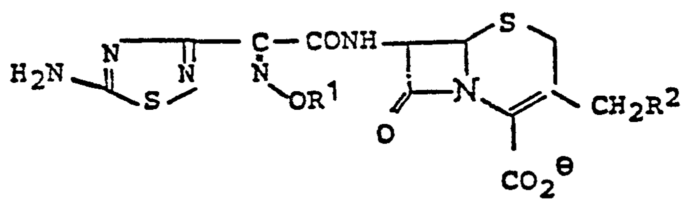

(I)

et sels d'addition avec des acides physiologiquement acceptables de ceux-ci, dans laquelle formule

$R^1$ représente un groupe alkyle en $C_1$-$C_2$ ;

$R^2$ représente un reste pyridinium

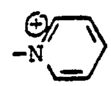

,

qui est substitué par deux groupes alkyle en position ortho, qui sont cyclisés en un noyau tri- à pentaméthylène, dans lequel un atome de carbone nucléaire peut être remplacé par un atome d'oxygène, ou un reste 1-quinoléinium ou un reste 2-isoquinoléinium,

et dans laquelle le groupe $OR^1$ est en position syn.

2. Procédé pour la préparation de composés de formule I et de leurs sels d'addition avec des acides physiologiquement acceptables, caractérisé en ce que

a) on fait réagir un composé de formule générale II

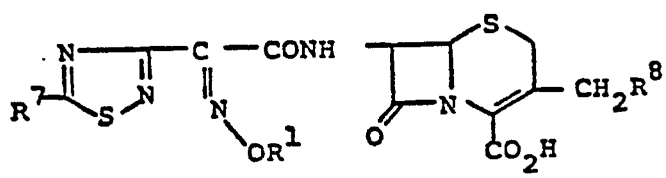

(II)

ou ses sels ou un dérivé réactif du composé II dans lequel $R^1$ a la signification ci-dessus et $R^7$ représente un groupe amino ou amino-protégé et $R^8$ représente un groupe échangeable par les dérivés de pyridine, de quinoléine ou d'isoquinoléine qui correspondent aux groupes $R^2$ de la formule I, avec ces dérivés de pyridine, quinoléine ou isoquinoléine et

α) on élimine un groupe protecteur éventuellement présent et

β) si nécessaire, on convertit le produit obtenu en un sel physiologiquement acceptable d'addition avec un acide, ou

b) on fait réagir un composé de 7-amino-céphème de formule générale III

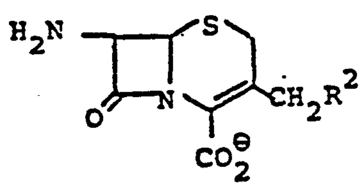

(III)

ou ses sels d'addition avec des acides, dans lesquels $R^2$ a la signification ci-dessus et le groupe amino pouvant être également sous la forme d'un dérivé réactif, avec l'acide 2-(5-amino-1,2,4-thiadiazole-3-yl)-2-syn-oximinoacétique de formule générale IV

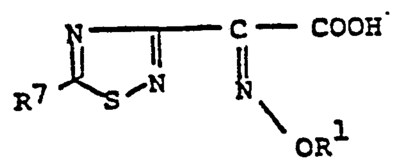

(IV)

dans laquelle $R^1$ et $R^7$ ont les significations indiquées ci-dessus ou avec un dérivé activé de ce composé, et

α) on élimine un groupe protecteur éventuellement présent et

β) si nécessaire, on convertit le produit obtenu en un sel d'addition physiologiquement acceptable avec un acide.

3. Procédé selon la revendication 2, caractérisé en ce que l'échange nucléophile du substituant $R^8$ a lieu en présence d'ions de sels neutres, en particulier d'ions iodure ou thiocyanate.

4. Procédé selon la revendication 2, caractérisé en ce que l'échange nucléophile du substituant $R^8$ a lieu en présence de la base du groupe $R^2$ et de triméthyliodosilane.

5. Préparations pharmaceutiques efficaces contre des infections bactériennes, caractérisées par une teneur en dérivés céphème de formule générale I.

6. Procédé pour la préparation de compositions pharmaceutiques efficaces contre des infections bactériennes, caractérisé en ce qu'on met un dérivé céphème de formule générale I sous une forme d'administration pharmaceutiquement appropriée, éventuellement avec des véhicules, diluants ou substances tampons pharmaceutiquement appropriés.

7. Dérivé céphème de formule générale I selon la revendication 1, pour la lutte contre des infections bactériennes.

**Revendications** (pour l'Etat contractant AT)

1. Procédé pour la préparation de dérivés céphème de formule I

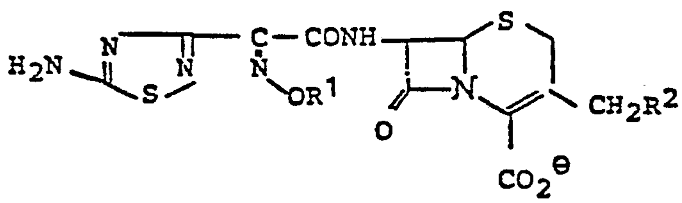

(I)

et de leurs sels d'addition avec des acides physiologiquement acceptables, dans laquelle formule

$R^1$ représente un groupe alkyle en $C_1$-$C_2$,

$R^2$ représente un reste pyridinium

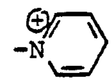

,

qui est substitué par deux groupes alkyle en position ortho qui sont cyclisés en un noyau tri- à pentaméthylène, dans lequel un atome de carbone nucléaire peut être remplacé par un atome d'oxygène, ou un reste 1-quinoléinium ou un reste 2-isoquinoléinium,

et dans laquelle le groupe $OR^1$ est en position syn, caractérisé en ce que

a) on fait réagir un composé de formule générale II

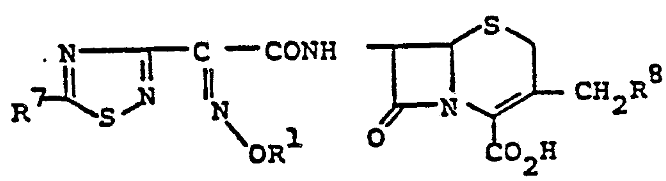

(II)

ou ses sels ou un dérivé réactif du composé II dans lequel $R^1$ a la signification ci-dessus et $R^7$ représente un groupe amino ou amino-protégé et $R^8$ représente un groupe échangeable par les dérivés de pyridine, de quinoléine ou d'isoquinoléine qui correspondent aux groupes $R^2$ de la formule I, avec ces dérivés de pyridine, quinoléine ou isoquinoléine et

α) on élimine un groupe protecteur éventuellement présent et

β) si nécessaire, on convertit le produit obtenu en un sel physiologiquement acceptable d'addition avec un acide, ou

b) on fait réagir un composé de 7-amino-céphème de formule générale III

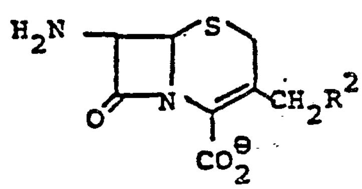

(III)

ou ses sels d'addition avec des acides, dans lesquels $R^2$ a la signification ci-dessus et le groupe amino pouvant être également sous la forme d'un dérivé réactif, avec l'acide 2-(5-amino-1,2,4-thiadiazole-3-yl)-2-syn-oximinoacétique de formule générale IV

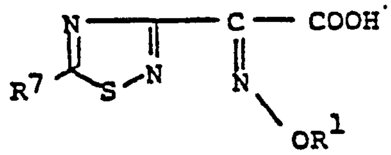

(IV)

dans laquelle $R^1$ et $R^7$ ont les significations indiquées ci-dessus ou avec un dérivé activé de ce composé, et

α) on élimine un groupe protecteur éventuellement présent et

β) si nécessaire, on convertit le produit obtenu en un sel d'addition physiologiquement acceptable avec un acide.

2. Procédé selon la revendication 1, caractérisé en ce que l'échange nucléophile du substituant $R^8$ a lieu en présence d'ions de sels neutres, en particulier d'ions iodure ou thiocyanate.

3. Procédé selon la revendication 1, caractérisé en ce que l'échange nucléophile du substituant $R^8$ a lieu en présence de la base du groupe $R^2$ et de triméthyliodosilane.